# EUROPÄISCHE PATENTANMELDUNG

(11) **EP 1 344 816 A1**
(43) Veröffentlichungstag der Anmeldung: **17.09.2003**
(21) Anmeldenummer: 03003441.7
(22) Anmeldetag: 14.02.2003
(51) Int. Cl.: C12M 1/00, C12M 1/107, C12P 5/02

(54) **Verfahren und Vorrichtung zur Eindickung von Gärgut aus der Vergärung von Abfällen**

(30) Priorität: 22.02.2002 DE 10207518
(71) Anmelder: LINDE-KCA-Dresden GmbH, 01277 Dresden (DE)
(72) Erfinder: Schiffmann, Manuel, 2503 Biel (CH)

(57) **Zusammenfassung**

Zur Eindickung von Gärgut aus der Vergärung von Abfällen wird vorgeschlagen das einem Fermenter 7 entnommene Gärgut ohne Zwischenschaltung einer mechanischen Entwässerung direkt einer Vakuumtrocknung 2 zu unterziehen.

## Beschreibung

Die Erfindung betrifft ein Verfahren zur Vergärung von biologisch abbaubare Bestandteile enthaltenden Abfällen in einem Fermenter, wobei das dem Fermenter entnommene Gärgut eingedickt wird, sowie eine Vorrichtung zur Durchführung des Verfahrens.

Eine zunehmend wichtige Alternative zur herkömmlichen Entsorgung von kommunalen und gewerblichen Abfällen durch Deponierung oder Verbrennung stellt die biologische Aufbereitung von biologisch abbaubare Bestandteile enthaltenden Abfällen dar. Neben der Kompostierung kommt hier der Vergärung eine immer größer werdende Bedeutung zu. Dabei wird aus den Abfällen in einem unter Luftabschluss gehaltenen Fermenter durch anaerobe Mikroorganismen ein hauptsächlich aus Kohlendioxid und Methan bestehendes Biogas erzeugt, welches energetisch genutzt werden kann. Übrig bleibt nicht in Biogas umgesetztes Gärgut, das einer Weiterverarbeitung zugeführt werden muss. Üblicherweise wird das dem Fermenter entnommene Gärtgut zunächst mechanisch beispielsweise mittels einer Schneckenpresse entwässert und das dabei anfallende Presswasser anschließend weiter verarbeitet. Das bei der mechanischen Entwässerung übrigbleibende Pressgut wird unter Zugabe von Strukturmaterial einer Kompostierung zugeführt.

Die bisher übliche Methode der mechanischen Entwässerung mittels Schneckenpressen macht eine aufwändige Überwachung der Schneckenpresse notwendig. Aufgrund schwankender Materialzusammensetzung hinsichtlich Konsistenz und Abbaugrad sind Modifikationen häufig erforderlich.
Außerdem weisen die Schneckenpressen einen hohen Verschleiß auf, was Materialkosten sowie einen erhöhten Zeit -und Personalaufwand für den Wechsel von Schneckenpressenteilen erfordert. Ein weiteres Problem bei der bisherigen Methode ist ein großer Anfall an Presswasser. Eine Nutzung dieses Presswassers ist problematisch, da eine Lagerung aufgrund eines hohen Sandanteils schwierig ist. Es müssten Rührwerke installiert werden, was zusätzliche Betriebskosten verursacht. Aufgrund kurzer Ausbringzeiten ist auch ein Einsatz in der Landwirtschaft nur begrenzt möglich. Außerdem ist das Presswasser bei Landwirten unbeliebt, da wegen des hohen Sandanteils Probleme bei Pumpen auftreten können.

Insgesamt ist die bekannte Methode der Eindickung von Gärgut aufwendig und störanfällig. Insbesondere kann die mechanische Entwässerung Probleme bereiten. Sie macht regelmäßig teure Verschleißreperaturen notwendig und bedingt einen hohen Stromverbrauch. Der notwendige Verfahrensschritt der Eindickung des Gärgutes kann die Wirtschaftlichkeit und Zuverlässigkeit einer Vergärungsanlage als Ganzes entscheidend beeinflussen.

Der vorliegenden Erfindung liegt daher die Aufgabe zugrunde, ein Verfahren der Eingangs genannten Art sowie eine Vorrichtung zur Durchführung des Verfahrens so auszugestalten, dass auf wirtschaftliche Weise eine zuverlässige Eindickung des Gärguts erreicht wird.

Diese Aufgabe wird verfahrensseitig erfindungsgemäß dadurch gelöst, dass das Gärgut zur Eindickung ohne vorherige Entwässerung direkt unter Vakuum getrocknet wird.

Auf diese Weise kann der Verfahrensschritt der mechanischen Entwässerung mit beispielsweise einer Schneckenpresse eingespart werden. Das Risiko von Betriebsstörungen in Folge von Fehlfunktionen oder Verschleißerscheinungen der Schneckenpresse wird dadurch beseitigt.

Das dem Fermenter entnommene Gärgut weist vor der Trocknung üblicherweise einen Trockensubstanzgehalt von ca. 15- ca. 30% auf. Durch die Vakuumtrocknung wird der Trockensubstanzgehalt vorzugsweise auf ca. 30- ca. 45% erhöht. Das bei der Vakuumtrocknung anfallende feststofffreie Kondensat wird zweckmäßigerweise vom verbleibenden Trockengut abgetrennt. Das verbleibende Trockengut kann mit Strukturmaterial vermischt werden und das so entstehende Mischgut wird bevorzugt einer Kompostierung zugeführt. Das entstehende Mischgut, das der Kompostierung zugeführt wird, soll einen Trockensubstanzgehalt von mindestens 35% haben.

Gemäß einer besonders bevorzugten Ausführungsform der Erfindung wird die Trocknung bei einem Vakuum von ca. -0,6 bis ca. -0,9 bar durchgeführt. Außerdem wird bei der Trocknung vorzugsweise eine Temperatur von ca. 70 bis ca. 80°C eingestellt.

Um den Trocknungsvorgang weiter zu beschleunigen wird außerdem Empfohlen, das Gärgut während der Trocknung zu bewegen. Hierzu kann beispielsweise eine drehende Spirale vorgesehen sein.

Die Erfindung betrifft femer eine Vorrichtung zur Vergärung von biologisch abbaubare Bestandteile enthaltenden Abfällen mit einem Fermenter und einer dem Fermenter nachgeschalteten Eindickeinrichtung zum Eindicken des Gärgutes.

Vorrichtungsseitig wird die gestellte Aufgabe dadurch gelöst, dass die Eindickungseinrichtung als Vakuumtrockner ausgebildet ist, welcher ohne Zwischenschaltung einer Entwässerungseinrichtung dem Fermenter nachgeschaltet ist.

Der Vakuumtrockner ist zweckmäßigerweise trommelförmig mit einer Zuführung für das Gärgut und einer Abführung für getrocknetes Gärgut am Ende der Trommel ausgebildet. Im Inneren des trommelförmigen Vakuumtrockners ist eine drehbare Spirale zum bewegen und axialen Fördern des Gärguts angeordnet. Ferner weist der Vakuumtrockner eine Heizeinrichtung zum Aufheizen des Trommelinnenraumes und einen Anschluss für eine Vakuumpumpe auf.

Der Vakuumpumpe ist vorzugsweise ein Kondensator zum Abtrennen von Kondensat von im Vakuumtrockner entstehenden Dampfbrüden vorgeschaltet. Zum Aufheizen des Vakuumtrockners weist die Heizeinrichtung vorteilhafterweise am Trommelmantel angeordnete Wärmetauscher auf, wobei der Außenumfang der Spirale einen Abstand von weniger als 5 cm, vorzugsweise 1-3 cm, von den Wärmetauschem hat.
Auf diese Weise werden die Wärmetauscher durch Bewegung der Spirale kontinuierlich gereinigt und ein Ankleben von Gärgut an den beheizten Trommelwänden verhindert. Dadurch wird ein zuverlässiger Wärmeaustausch auch bei längeren Betriebszeiten des Trockners gewährleistet.

Eine besonders bevorzugte Ausgestaltung der Erfindung sieht vor, dass zwischen dem Fermenter und dem Vakuumtrockner ein Entnahmebehälter zwischengeschaltet ist, der mittels eines Entnahmeschiebers und mittels eines Ablaufschiebers vom Vakuumtrockner trennbar ist. An den Entnahmebehälter sind außerdem ein Kompressor und eine Vakuumpumpe angeschlossen. Beim Betrieb der Anlage wird zunächst das dem Fermenter entnommene Gärgut in den Entnahmebehälter eingefüllt. Der Entnahmebehälter wird mittels der Vakuumpumpe mit Unterdruck beaufschlagt. Nach dem Befüllen des Entnahmebehälters wird der Entnahmeschieber geschlossen und der Ablaufschieber geöffnet. Der Kompressor sorgt nun dafür, dass das Gärgut in den Vakuumtrockner gedrückt wird.

### Die Erfindung bietet eine ganze Reihe von Vorteilen:

Im Gegensatz zur bisherigen Methode der Eindickung von Gärgut aus Fermentern wird mit der Erfindung aufgrund des Verzichts auf eine mechanische Entwässerung mittels einer Schneckenpresse eine sehr hohe Betriebssicherheit gewährleistet. Die erfindungsgemäße Eindickungsvorrichtung ist ohne komplizierte Mechanik aufgebaut und arbeitet mit geringen Antriebsleistungen, wodurch die Gefahr von Betriebsstörungen deutlich reduziert wird. Sie ist wenig verschleißanfällig und ein Verklemmen oder Aufstauen von Gärgut wird zuverlässig verhindert. Aufwendige Arbeiten für einen Wechsel von Verschleißteilen, wie z. B. Schneckenteilen, Körben etc. können aufgrund des Verzichts auf eine mechanische Entwässerung entfallen. Auch die Energiekosten sind sehr viel geringer als bei der Verwendung einer mechanischen Entwässerung, da der Vakuumtrockner nur einen sehr kleinen Stromverbrauch hat.

### Die Erfindung bietet aber auch verfahrenstechnische Vorteile:

Durch Einstellung der Heizleistung (d. h. der pro Charge zugeführten Wärmemenge) des Vakuumtrockners kann der Trockensubstanzgehalt des Trockengutes im Voraus gewählt werden. Außerdem ist eine Mischung des Trockengutes mit Strukturmaterial für die nachfolgende Kompostierung einfacher herstellbar, da nur zwei Fraktionen gemischt werden müssen, nämlich das Trockengut und das Strukturmaterial, während beim bisherigen Einsatz einer zweistufigen Entwässerung drei Fraktionen gemischt werden mussten, nämlich Strukurmaterial, Pressgut und Konzentrat aus der zweiten Stufe. Von Vorteil ist ferner, dass das mit Strukturmaterial gemischte Trockengut praktisch geruchlos ist.

Im Folgenden werden der bisherige Stand der Technik sowie ein Ausführungsbeispiel der Erfindung anhand von Figuren näher erläutert.

Es zeigen:
- Figur 1: ein Fließschema der Gärguteindickung nach dem Stand der Technik.
- Figur 2: ein Fließschema der Gärguteindickung nach einem Ausführungsbeispiel der Erfindung.
- Figur 3: eine schematische Darstellung einer Anlagenanordnung nach einem Ausführungsbeispiel der Erfindung.

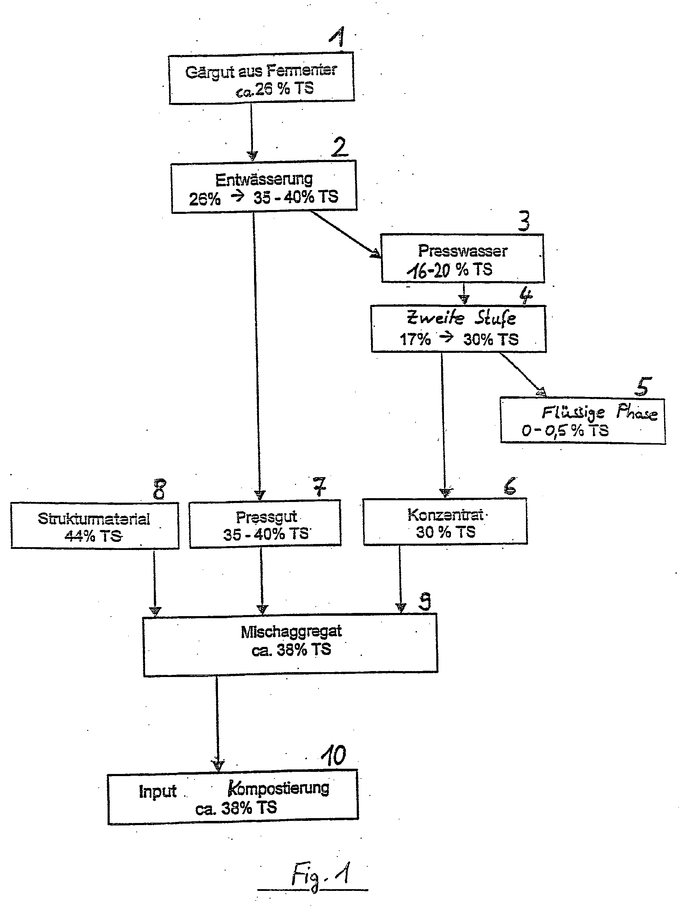

Nach dem in Figur 1 dargestellten Stand der Technik wird Gärgut 1 mit einem Trockensubstanzgehalt von ca. 26% aus dem Fermenter abgezogen und einer Entwässerung zugeführt. Dort wird das Gärgut auf mechanische Weise z. B. mittels einer Schneckenpresse zu einem Pressgut 7 mit einem Trockensubstanzgehalt von 35-40% eingedickt. Bei der mechanischen Entwässerung fällt Presswasser 3 mit einem Trockensubstanzgehalt von 16-20% an. Dieses Presswasser wird einer zweiten Stufe 4 unterzogen, bei der der Trockensubstanzgehalt von 17% auf 30% erhöht wird. Feststoffarmes Abwasser 5 wird aus der Anlage abgezogen, während Konzentrat 6 mit einem Trockensubstanzgehalt von 30% einem Mischaggregat 9 zugeführt wird. Im Mischaggregat 9 wird das Konzentrat 6 mit dem Pressgut 7 und einem einen Trockensubstanzgehalt von 44% aufweisenden Struckturmaterial gemischt. Das so entstehende Mischgut mit einem Trockensubstanzgehalt von ca. 38% wird schließlich einer Kompostierung zugeführt.

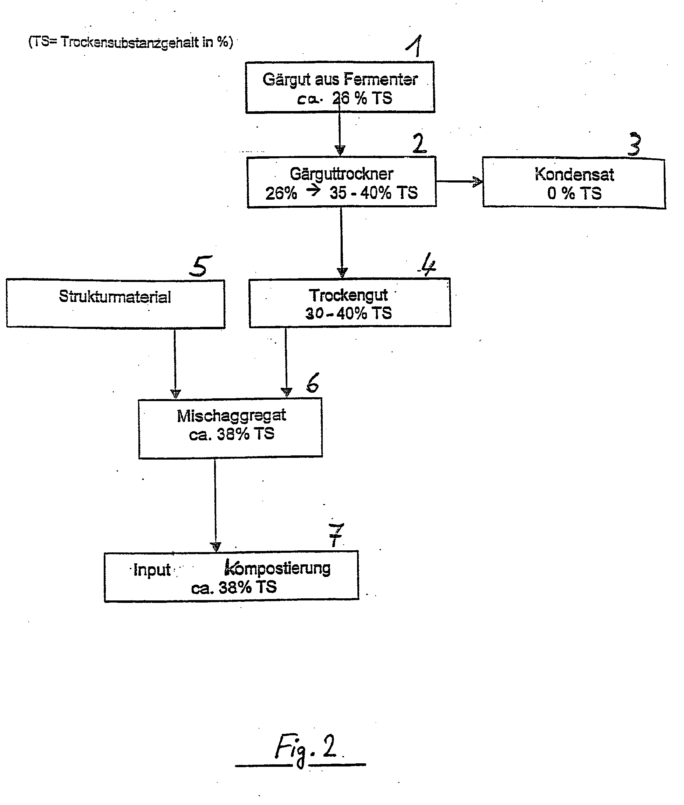

Im Vergleich zu der in Figur 1 dargestellten Gärguteindickung nach dem Stand der Technik kommt die Gärguteindickung nach dem in Figur 2 gezeigten Ausführungsbeispiel der Erfindung mit wesentlich weniger Verfahrensschritten aus. Das dem Fermenter entnommene Gärgut 1 mit einem Trockensubstanzgehalt von ca. 26% wird unmittelbar ohne Zwischenschaltung einer Entwässerung dem Vakuumtrockner 2 zugeführt. Im Vakuumtrockner 2 wird durch Aufheizung unter Vakuum das Gärgut auf einen Trockensubstanzgehalt von 30- 40% eingedickt. Dabei fällt feststofffreies Kondensat 3 an, das abgezogen werden kann. Das verbleibende Trockengut 4 mit einem Trockensubstanzgehalt von 30- 40% wird einem Mischaggregat 6 zugeführt. Dort wird das Trockengut mit Strukturmaterial zu einem Mischgut mit einem Trockensubstanzgehalt von 35-40% gemischt. Das Mischgut wird schließlich einer Kompostierung 7 zugeführt.

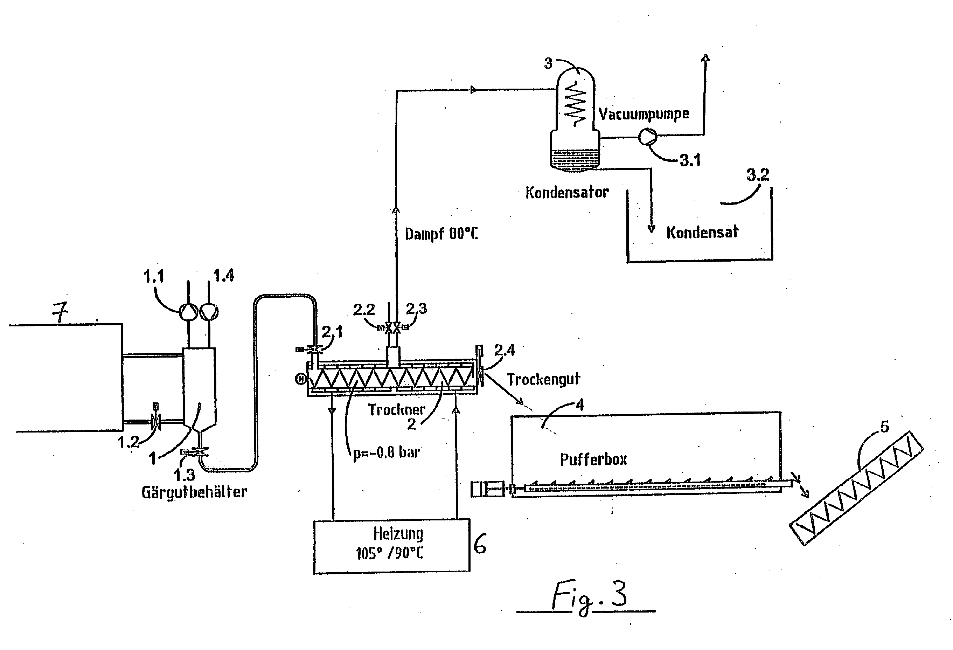

Beim Betrieb der in Figur 3 als Ausführungsbeispiel dargestellten Anlage zur Gärguttrockung wird zunächst der Entnahmebehälter 1 durch die Vakuumpumpe 1.1 mit Unterdruck (ca. 0,7 bar) beaufschlagt. Nach Erreichen des Unterdrucks wird der Entnahmeschieber 1.2 am Fermenter 7 geöffnet, so dass das Gärgut in den Entnahmebehälter 1 fließt. Nach Erreichen eines maximalen Füllstandes wird der Entnahmeschieber 1.2 geschlossen, wonach der Ablaufschieber 1.3 geöffnet wird. Mit dem Kompressor 1.4 wird nun im Entnahmebehälter Druck aufgebaut, so dass das Gärgut in den Vakuumtrockner 2 gefördert wird.

Der leere Vakuumtrockner 2 wird mit einem Inhalt aus dem Entnahmebehälter 1 beschickt. Da der Füllgrad des Entnahmebehälters 1 definiert ist, (wird immer gleichweit aufgefüllt), ist auch die Gärgutmenge im Vakuumtrockner 2 bestimmt. Der Füllgrad des Vakuumtrockners 2 beträgt ca. 60%. Am Vakuumtrockner 2 sind ein Befüllschieber2.1, ein Entlüftungsventil 2.2 und ein Vakuumventil 2.3 angebracht. Nach Schließen des Befüllschiebers 2.1 am Vakuumtrockner wird das Entlüftungsventil 2.2 geschlossen und das Vakuumventil 2.3 geöffnet. Sobald das gewünschte Vakuum (ca. -0,8 bar) erreicht ist, wird die Heizung gestartet. Hierzu werden z. B. am Innenmantel des Vakuumtrockners 2 angeordnete Wärmetauscher mit Heißwasser aus einer Heizanlage 6 beschickt. Nach einer Aufwärmzeit beginnt die Verdampfung bei einer Temperatur von ca. 75°C. Durch Vor- und Zurückdrehen der im Vakuumtrockner 2 angeordneten Spirale wird das Gärgut bewegt und werden die Wärmetauscher des Vakuumtrockners 2 kontinuierlich gereinigt. Aufgrund einer kleinen Drehzahl von ein bis zwei Umdrehungen pro Minute benötigt der Spiralenantrieb des Vakuumtrockners 2 eine elektrische Anschlussleistung von lediglich 1,5 kW (verbraucht ca. 0,5 kW). Da die Reibungsverluste gering sind, ist der Verschleiß an der Spirale und am Heizmantel minimal. Die Trocknung einer Charge dauert ca. 3 - 5 Stunden.

Die im Vakuumtrockner 2 entstehenden Dampfbrüden strömen in den Kondensator 3 und werden vor der Vakuumpumpe 3.1 abgekühlt. Das anfallende Kondensat wird im integrierten Auffangbehälter gesammelt und periodisch in einen Kondensatbehälter 3.2 abgepumpt.

Das Vakuum wird durch die Drehzahl der Vakuumpumpe 3.1 geregelt. Die Wäremzufuhr des Vakuumtrockners wird überwacht und registriert. Es ist eine Anlagensteuerung vorgesehen, an der der gewünschte Trockensubstanzgehalt (ca. 30 - 40%) eingegeben werden kann. Die Steuerung rechnet die zu verdampfende Menge sowie die benötigte Wärmemenge aus. Sobald die entsprechende Wärmemenge zugeführt worden ist, schließt das Vakuumventil 2.3 und das Entlüftungsventil 2.2 öffnet sich. Ein am Ende des Vakuumtrockners 2 angeordneter Entnahmeschieber 2.4 wird geöffnet. Durch Vorwärtsdrehen der Spirale wird das Trockengut aus dem Vakuumtrockner 2 gefördert, so dass es z. B. in eine Pufferbox 4 fällt, wo es zwischengelagert werden kann.

Gegebenfalls kann Strukturmaterial in die Pufferbox hinzugegeben werden oder in einer nachgeschalteten Steig-Mischspirale 5.

Eine Vergärungsanlage weist zweckmäßigerweise mindestens 2 Vakuumtrockner auf, die im kontinuierlichen Betrieb während 24 Stunden pro Tag arbeiten. Die Entleerung eines Vakuumtrockners kann zu einer beliebigen Zeit erfolgen.

## Patentansprüche

1. Verfahren zur Vergärung von biologisch abbaubare Bestandteile enthaltenden Abfällen in einem Fermenter, wobei das dem Fermenter entnommene Gärgut eingedickt wird, **dadurch gekennzeichnet, dass** das Gärgut zur Eindickung ohne vorherige Entwässerung direkt unter Vakuum getrocknet wird.

2. Verfahren nach Anspruch 1, **dadurch gekennzeichnet, dass** das Gärgut vor der Trocknung einen Trockensubstanzgehalt von ca. 15- ca. 30% und nach der Trocknung einen auf ca. 30- ca. 45% erhöhten Trockensubstanzgehalt aufweist.

3. Verfahren nach Anspruch 1 oder 2, **dadurch gekennzeichnet, dass** bei der Trocknung anfallendes, feststofffreies Kondensat vom verbleibenden Trockengut abgetrennt wird.

4. Verfahren nach Anspruch 3, **dadurch gekennzeichnet, dass** das verbleibende Trockengut mit Strukturmaterial vermischt wird und so entstehendes Mischgut einer Weiterbehandlung zugeführt wird.

5. Verfahren nach einem der Ansprüche 1 bis 4, **dadurch gekennzeichnet, dass** die Trocknung bei einem Vakuum von ca. -0,6 bis ca. -0,9 bar durchgeführt wird.

6. Verfahren nach einen der Ansprüche 1 bis 5, **dadurch gekennzeichnet, dass** bei der Trocknung eine Temperatur von ca. 70 bis ca. 80°C eingestellt wird.

7. Verfahren nach einem der Ansprüche 1 bis 6, **dadurch gekennzeichnet, dass** das Gärgut während der Trocknung bewegt wird.

8. Verfahren nach Anspruch 7, **dadurch gekennzeichnet, dass** das Gärgut mittels einer drehenden Spirale bewegt wird.

9. Vorrichtung zur Vergärung von biologisch abbaubare Bestandteile enthaltenden Abfällen mit einem Fermenter und einer dem Fermenter nachgeschalteten Eindickeinrichtung zum Eindicken des Gärgutes, **dadurch gekennzeichnet, dass** die Eindickungseinrichtung als Vakuumtrockner ausgebildet ist, welcher ohne Zwischenschaltung einer Entwässerungseinrichtung dem Fermenter nachgeschaltet ist.

10. Vorrichtung nach Anspruch 9, **dadurch gekennzeichnet, dass** der Vakuumtrockner trommelförmig mit einer Zuführung für das Gärgut und einer Abführung für getrocknetes Gärgut am Ende der Trommel ausgebildet ist, wobei im Innern des trommelförmigen Vakuumtrockners eine drehbare Spirale zum Bewegen und axialen Fördern des Gärguts angeordnet ist und der Vakuumtrockner eine Heizeinrichtung zum Aufheizen des Trommelinnenraumes und einen Anschluss an eine Vakuumpumpe aufweist.

11. Vorrichtung nach Anspruch 10, **dadurch gekennzeichnet, dass** der Vakuumpumpe ein Kondensator zum Abtrennen von Kondensat von im Vakuumtrockner entstehenden Dampfbrüden vorgeschaltet ist.

12. Vorrichtung nach Anspruch 9 oder 11, **dadurch gekennzeichnet, dass** die Heizeinrichtung am Trommelmantel angeordnete Wärmetauscher aufweist und der Außenumfang der Spirale einen Abstand von weniger als 5cm, vorzugsweise 1-3 cm, von den Wärmetauschern hat.
